# EUROPEAN PATENT SPECIFICATION

(11) **EP 3 400 038 B1**
(45) Date of publication and mention of the grant of the patent: **31.07.2024**
(21) Application number: 17700007.2
(22) Date of filing: 02.01.2017
(51) Int. Cl.: A61M 5/14, A61M 5/162

(54) **DRIP CHAMBER FOR ADMINISTERING ENTERAL NUTRITION OR ANOTHER MEDICAL FLUID**
TROPFKAMMER ZUR VERABREICHUNG EINES ENTERALEN NÄHRMITTELS ODER EINER ANDEREN MEDIZINISCHEN FLÜSSIGKEIT
CHAMBRE COMPTE-GOUTTES POUR L'ADMINISTRATION DE NUTRITION ENTÉRALE OU AUTRE FLUIDE MÉDICAL

(30) Priority: 04.01.2016 EP 16150022
(43) Date of publication of application: 14.11.2018
(73) Proprietor: Fresenius Kabi Deutschland GmbH, 61352 Bad Homburg (DE)
(72) Inventor: HADASCHIK, Roman, 99817 Eisenach (DE); HUTH, Matthias, 99831 Ifta (DE); SAUERBIER, Carsten, 34212 Melsungen (DE)
(74) Representative: Fresenius Kabi Deutschland GmbH
(86) International application number: PCT/EP2017/050005
(87) International publication number: WO 2017/118615

(56) References cited:
- EP-A1- 0 355 795
- EP-A1- 1 027 900
- CN-U- 204 275 157
- CN-U- 204 501 885
- DE-A1- 102006 041 414
- US-A- 5 735 826

## Description

The invention relates to a drip chamber for administering enteral nutrition or another medical fluid according to the preamble of claim 1 and to a method for producing a drip chamber for administering enteral nutrition or another medical fluid.

The invention in particular relates to a drip chamber for administering enteral nutrition or another medical fluid comprising a body forming a chamber and having an inlet end and an outlet end. A spike element is arranged at the inlet end of the body and forms an inlet for letting a medical fluid into the chamber. An outlet is arranged at the outlet end of the chamber and is connectable to a tube for letting a medical fluid out of the chamber.

Via the spike element the drip chamber may for example be connected to a connector of a container containing enteral nutrition , such as a flexible bag, an adapter for a glass bottle or the like. The spike element herein serves to for example pinch a membrane such, that a fluid connection between the container and the chamber is established to allow a fluid to flow into the drip chamber.

To connect the drip chamber to the connector of for example a container, a connection element is rotatably arranged on the spike element. The connection element allows for a releasable connection of the drip chamber to the connector, for example by establishing a screw connection between the connection element and the connector.

A drip chamber may, for example, have the purpose to allow gas (such as air) to rise out from a fluid so that it is not passed down-streams. The use of a drip chamber furthermore allows to estimate the rate at which a fluid is administered. In particular, for a fluid of given viscosity, drips from a hole of known size will have an identical volume, such that the number of drips in a minute for example can be counted to obtain an estimate of the flow rate. Within an enteral feeding set, the flow rate herein may for example be controlled by a clamp on a tube, effecting the flow resistance of the tube.

A drip chamber of this kind may be used for example for the administration of enteral feeding solutions or, generally, medical fluids into the gastro-intestinal tract of the patient such as lipids, drugs or another liquids and for this purpose may be part of an enteral feeding set. A drip chamber of this kind may also be used, for example, for transfusion or infusion of medical fluids.

A drip chamber known from WO 2008/058656 A1 comprises a cap element connected to a body of the drip chamber, the cap element forming fastening elements for fastening a connector of a medical container to the drip chamber. US 5 735 826 A and EP 0 355 795 A1 disclose drip chambers according to the preamble of claim 1.

Within conventional enteral drip chambers, a spike element and a cap element for closing off the body at its inlet end are formed as separate pieces. This makes it necessary to connect the spike element to the cap element for example by means of a glue connection between the two pieces, for which it must be ensured that a fluid-tight connection is established. Overall, the production of the spike element and the cap element by separate pieces increases the number of parts to be produced, increases the required steps for completing the drip chamber and hence may increase costs of the drip chamber.

It is an object to provide a drip chamber and a method for producing a drip chamber which allow for an easy, reliable, cost-efficient production of the drip chamber, including the mounting of the rotatable connection element on the spike element.

This object is achieved by means of the drip chamber comprising the features of claim 1.

Accordingly, the drip chamber comprises a cap element connected to the body for closing off the body at the inlet end, the cap element being integrally formed with the spike element.

Hence, the cap element and the spike element are integrally formed in one piece. Thus, the number of parts is reduced in that a single element is formed for closing off the body at the inlet end and for providing a fluid connection to for example a container containing a medical fluid to be administered to a patient.

The cap element together with the spike element may for example be formed from plastics, for example using an injection molding technique. Herein, the cap element together with the spike element may for example be formed from a material having an increased rigidity as compared to the material of the body. In particular, the body may be formed from a transparent, rather soft plastics material, whereas the cap element together with the spike element is formed from a comparatively stiff plastics material.

The body may for example have a substantially cylindrical shape extending longitudinally between its inlet end and its outlet end. The cap element in this regard may have a circular shape for closing off the body at its inlet end.

The connection element serves to provide a releasable connection between the drip chamber and a connector of for example a medical container containing a enteral nutrition or another medical fluid to be administered to a patient. For example, the drip chamber may serve for the enteral or parenteral feeding, and accordingly the container may contain an enteral or parenteral feeding solution, for example a nutritional solution.

The connection element may for example have the shape of a coupling nut having an opening and a screw thread arranged therein, such that a screw connection may be established between the drip chamber and the connector of the container by means of the connection element.

To allow for establishing a connection between the drip chamber and the connector of for example a container containing enteral nutrition, the connection element is rotatably arranged on the spike element. The connection element hence may be rotated with respect to the spike element in order to for example screw the connection element onto an associated screw thread of the connector. Herein, the connection element is axially fastened to the spike element for example by a positive locking connection such that the connection element may be rotated with respect to the spike element, but is axially fixed to the spike element, thus allowing to hold the spike element axially in place with respect to the connector.

The positive locking connection may for example be established by means of one or multiple detent elements of the connection element engaging with a circumferential protrusion element of the spike element. The one or the multiple detent elements are elastically deformable such that they can radially give way when the connection element is placed on the spike element. For arranging the connection element on the spike element, the connection element can be slid onto the spike element from a far end of the spike element opposite to the cap element, wherein the connection element is slid along the spike element until the detent elements of the connection element snap into place with the circumferential protrusion element of the spike element.

The protrusion element extends circumferentially about a shaft portion of the spike element, the connection element engaging with the protrusion element when it is placed on the spike element.

The connection element and the integral piece of the cap element and the spike element may be fabricated in separate fabrication steps as separate pieces. In this case the connection element is placed, after fabrication, on the spike element in order to establish a positive locking connection between the connection element and the spike element. In a mounted state the connection element is then axially fastened on the spike element and in particular is held axially fixed in between the protrusion element of the spike element and the cap element, but is rotatable with respect to the spike element in order to allow for establishing a connection between the drip chamber and a connector of for example a fluid container.

In another embodiment the connection element may be fabricated together with the spike element and the cap element in a generative fabrication process, for example utilizing a sintering technique or two-component (2K) injection molding technique. The connection element and the integral piece of the cap element and the spike element hence are formed in a single fabrication step, during which the connection element and the integral piece of the spike element and the cap element are formed as separate pieces for example in a dedicated molding tool such that the connection element, after fabrication, is rotatable with respect to the spike element.

The cap element is fixedly connected to the body of the drip chamber and serves to close off the body at its inlet end. For this, the cap element forms a fluid tight connection in that it rests with a flange portion on an abutment portion of the body. The cap element may for example be glued or welded to the body such that a fixed connection between the cap element and the body is established.

The cap element being integrally formed with the spike element comprises an opening which is in fluid connection with a flow channel of the spike element. Through the opening fluid may flow from the flow channel of the spike element into the chamber of the body at a controlled rate for administration to a patient.

The object is also achieved by a method according to claim 10, i.e. for producing a drip chamber for administering enteral nutrition or another medical fluid, the method inter alia comprising:
- providing a body forming a chamber and having an inlet end and an outlet end, an outlet being arranged at the outlet end and being connectable to a tube for letting a medical fluid out of the chamber, and
- arranging a spike element at the inlet end of the body, the spike element forming an inlet end for letting a medical fluid into the chamber, a connection element being rotatably arranged on the spike element for releasably connecting the drip chamber to a connector.

Herein, the spike element is integrally formed with a cap element for closing off the body at the inlet end.

The spike element may be integrally formed with the cap element using for example an injection molding technique.

Herein, the connection element may be formed as a separate piece and may be arranged, in a separate mounting step, on the spike element in order to establish a positive locking connection between the spike element and the connection element for axially fastening the connection element on the spike element.

In another embodiment, the connection element may be formed together with the integral piece of the spike element and the cap element applying a generative fabrication process such as a sintering technique, for example a laser sintering, or a two-component injection molding technique.

The advantages and advantageous embodiments described above for the drip chamber generally apply also to the method such that it shall be referred to the above.

The idea underlying the invention shall subsequently being described in more detail with regard to the embodiments shown in the figures. Herein:
- Fig. 1: shows a schematic view of an enteral feeding set for administering an enteral nutritional solution to a patient, the enteral feeding set employing a drip chamber;
- Fig. 2: a sectional view of an embodiment of a drip chamber having a cap element and a spike element formed in one piece;
- Fig. 3: a perspective view of the cap element and the spike element, with a connection element being arranged on the spike element; and
- Fig. 4: a schematic top view of the spike element, showing the cross-sectional contour of the spike element.

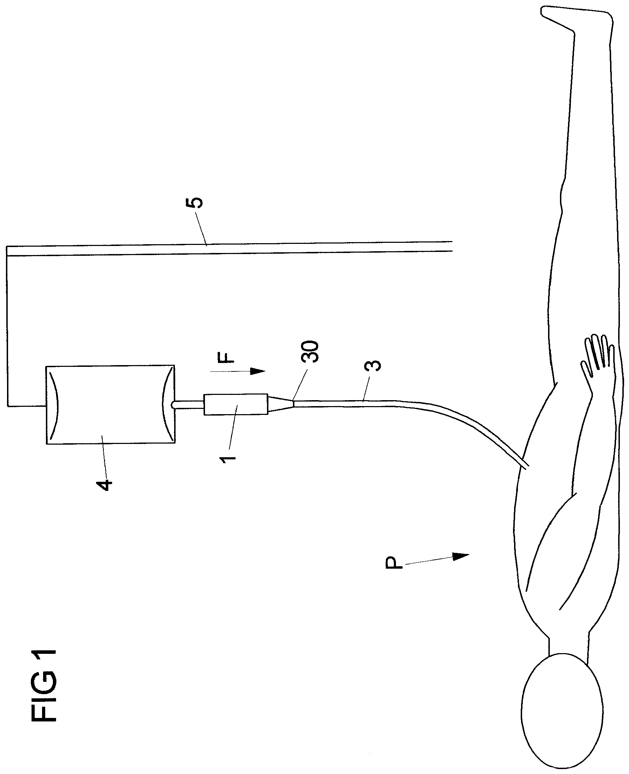

Fig. 1 shows a schematic view of an enteral feeding set for administering enteral nutrition or another medical fluid out of a container 4, for example in the shape of a flexible bag, to a patient P. The enteral feeding set set comprises a drip chamber 1 and a tube 3 connected at an end 30 to the drip chamber 1 for administering the medical fluid out of the container 4 in a flow direction F to the patient P. The container 4 may for example be arranged on a stand 5 at a bed side of the patient P in a hospital environment.

The drip chamber 1 serves in particular to allow gas, for example air, to rise out of the medical fluid to prevent gas to be transported down-streams towards the patient P. Furthermore, the drip chamber 1 may comprise a membrane for filtering the medical fluid before it is delivered to the patient P.

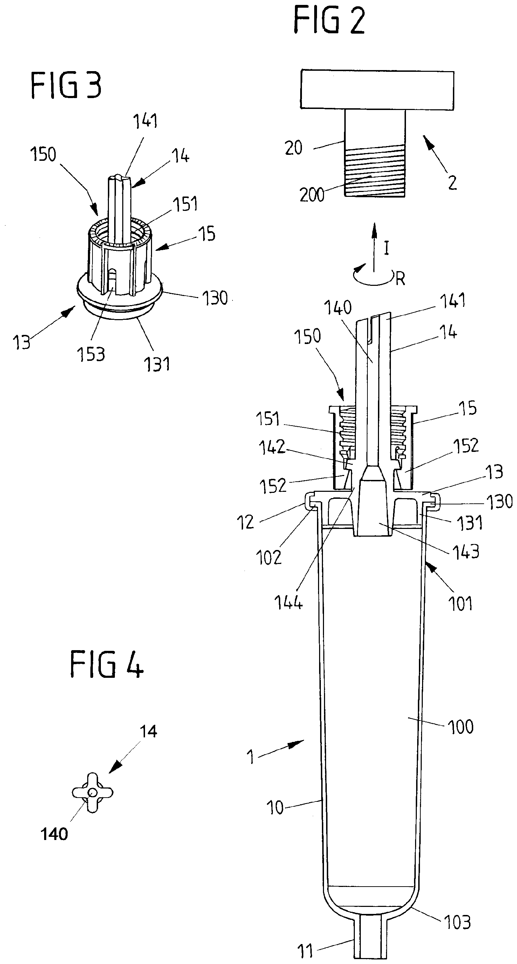

Fig. 2 shows a view of a drip chamber 1 as it may be employed, for example, for the enteral nutrition. The drip chamber 1 comprises a body 10 made for example from a rather soft, transparent plastic material and having an inlet end 101 and an outlet end 103. The body 10 forms a chamber 100 therein for receiving a medical fluid for example from a medical container 4 via the inlet end 101 and passing the fluid along to the tube 3 connected to an outlet 11 at the outlet end 103 of the body 10.

The body 10 is closed off at its inlet end 101 by a cap element 13 integrally formed with a spike element 14. The cap element 13 together with the spike element 14 may for example be formed from plastics, for example using an injection molding technique, and may be fabricated from a comparatively rigid material, the cap element 13 hence having an increased rigidity as compared to the body 10.

The cap element 13 forms a fluid-tight connection with the body 10 and for this, with an insertion portion 131, is partially inserted into the body 10 and rests, with a flange portion 130, on a circumferential abutment portion 102 at the inlet end 101 of the body 10. The abutment portion 102 is formed at the upper circumferential edge of the body 10 by forming a radially protruding flange, which is in abutment with the circumferential flange portion 130 of the cap element 13.

The cap element 13 is connected to the body 10 for example by gluing, overmoulding or by welding, wherein the connection is secured by means of a ring element 12 at least partially surrounding the flange portion 130 of the cap element 13 and the abutment portion 102 of the body 10.

The spike element 14 protrudes from the cap element 13 along an insertion direction I in which the spike element 14 may be inserted into a connector 2 of for example a medical container 4 such as an enteral feeding bag or the like. The spike element 14 forms a flow channel 140 longitudinally extending within the spike element 14 and being in fluid connection with an opening 143, the flow channel 140 opening into the chamber 100 of the body 10 such that fluid may flow through the flow channel 140 of the spike element 14 and may enter into the chamber 100 of the body 10.

At its far end 141 opposite the cap element 13 the spike element 14 has a slanted face forming a peak allowing to pinch for example a membrane or the like arranged on the connector 2.

A connection element 15 in the shape of a coupling nut is arranged on the spike element 14 and is axially fastened to the spike element 14 via detent elements 152 engaging with a circumferential protrusion element 142 extending about a shaft portion 144 of the spike element 14.

The connection element 15 is axially fixed on the spike element 14, but is rotatable about a rotational direction R with respect to the spike element 14. The connection element 15, within an opening 150, comprises a screw thread 151 by which it can be screwed onto a corresponding screw thread 200 on a shaft 20 of the connector 2 such that a screw connection between the drip chamber 1 and the connector 2 can be established. In a connected state, the connector 2 with its shaft 20 engages with the opening 150 of the connection element 15, the connection element 15 being screwed onto the shaft 20 and thus holding the spike element 14 in position relative to the connector 2.

The spike element 14 comprises, as schematically illustrated in Fig. 4, a non-rotationally symmetric contour corresponding to a complementary contour of an insertion opening of the shaft 20 of the connector 2. When inserting the spike element 14 into the connector 2, hence, the spike element 14 is rotationally fixed with respect to the connector 2. To establish the screw connection between the connection element 15 and the connector 2 the connection element 15 can be rotated in the rotational direction R with respect to the shaft 20 of the connector 2 without rotating the spike element 14 with respect to the connector 2 such that the screw thread 151 within the opening 115 of the connection element 15 comes into engagement with the screw thread 200 on the shaft 20 of the connector 2.

The integral piece of the cap element 13 and the spike element 14 on the one hand and the connection element 15 on the other hand may be formed as separate pieces for example by injection molding. For arranging the connection element 15 on the spike element 14 the connection element 15 in this case may be placed on the spike element 14 via the far end 141 and may be slid down the spike element 14 until the connection element 15 with its detent elements 152 snaps into engagement with the protrusion element 142 extending about the shaft portion 144 of the spike element 14.

To establish the connection between the connection element 15 and the spike element 14, the detent elements 152 are separated from one another by means of longitudinally extending slits 153 (see Fig. 3) such that the detent elements 152 can be elastically deformed when running up the protrusion element 142 and may elastically snap into engagement with the protrusion element 142 once they have passed the protrusion element 142 when sliding the connection element 15 onto the spike element 14.

In the mounted state the connection element 15 with its detent elements 152 comes to lie in between the protrusion element 142 of the spike element 14 and the cap element 13 such that the connection element 15 is axially fastened with respect to the spike element 14, but at the same time is rotatable along the rotational direction R with respect to the spike element 14.

In another embodiment the connection element 15 and the integral piece of the cap element 13 and the spike element 14 may be fabricated in a single fabrication process using for example a sintering technique, such as a laser sintering technique, or a two-component injection molding technique. Using such a generative fabrication technique, the connection element 15 on the one hand and the integral piece of the cap element 13 and the spike element 14 on the other hand may be formed as separate pieces, but in a single fabrication step, hence easing the fabrication of the drip chamber 1.

A drip chamber of the kind described herein may be used for example on an enteral feeding set for the enteral feeding, but may also be used for example on a tube set for the infusion or the transfusion. This however is not limiting. Generally, a drip chamber as described herein may be used for completely different purposes for administering a medical fluid to a patient.

### List of reference numerals

- 1: Drip chamber
- 10: Body
- 100: Chamber
- 101: Inlet end
- 102: Abutment portion
- 103: Outlet end
- 11: Outlet
- 12: Ring element
- 13: Cap element
- 130: Flange portion
- 131: insertion portion
- 14: Spike
- 140: Flow channel
- 141: End
- 142: Ring protrusion
- 143: Opening
- 144: Shaft portion
- 15: Coupling nut
- 150: Opening
- 151: Screw thread
- 152: Detent element
- 153: Slit
- 2: Connector
- 20: Shaft
- 200: Screw thread
- 3: Tube
- 30: End
- 4: Container (flexible bag)
- 5: Stand
- F: Flow direction
- I: insertion direction
- P: Patient
- R: Rotational direction

## Claims

1. A drip chamber (1) for administering enteral nutrition or another medical fluid, comprising:
- a body (10) forming a chamber (100) and having an inlet end (101) and an outlet end (103),
- a spike element (14) arranged at the inlet end (101) of the body (10) and forming an inlet (20) for letting a medical fluid into the chamber (100),
- an outlet (11) arranged at the outlet end (103) and being connectable to a tube (3) for letting a medical fluid out of the chamber (100), and
- a connection element (15) rotatably arranged on the spike element (14) for releasably connecting the drip chamber (1) to a connector (2),
**characterized by**
a cap element (13) connected to the body (10) for closing off the body (10) at the inlet end (101), the cap element (13) being integrally formed with the spike element (14), and wherein the connection element (15) comprises at least one elastically deformable detent element (152) such that the connection element can be slid onto the spike element from a far end of the spike element opposite to the cap element, and along the spike element until the at least one detent element of the connection element snaps into place with a protrusion element (142) of the spike element (14), the protrusion element (142) extending circumferentially about a shaft portion (144) of the spike element (14).

2. The drip chamber (1) according to claim 1, **characterized in that** the cap element (13) is integrally formed with the spike element (14) by injection molding.

3. The drip chamber (1) according to claim 1 or 2, **characterized in that** the connection element (15) is shaped as a coupling nut having an opening (150) and a screw thread (151) arranged therein.

4. The drip chamber (1) according to one of claims 1 to 3, **characterized in that** the connection element (15) is axially fastened to the spike element (14) by a positive locking connection.

5. The drip chamber (1) according to one of the preceding claims, **characterized in that** the connection element (15) is fabricated together with the spike element (14) and the cap element (13) by a generative fabrication process.

6. The drip chamber (1) according to one of the preceding claims, **characterized in that** the connection element (15) is fabricated together with the spike element (14) and the cap element (13) by a sintering technique or by a two-component injection molding technique.

7. The drip chamber (1) according to one of the preceding claims, **characterized in that** the cap element (13) comprises a flange portion (102) resting on an abutment portion (102) of the body (10) for forming a fluid-tight connection between the cap element (13) and the body (10).

8. The drip chamber (1) according to one of the preceding claims, **characterized in that** the cap element (13) is connected to the body (10) by gluing or welding.

9. The drip chamber (1) according to one of the preceding claims, **characterized in that** the cap element (13) comprises an opening (143) being in fluid connection with a flow channel (140) of the spike element (14).

10. A method for producing a drip chamber (1) for administering enteral nutrition or another medical fluid, comprising:
- providing a body (10) forming a chamber (100) and having an inlet end (101) and an outlet end (103), an outlet (11) being arranged at the outlet end (103) and being connectable to a tube (3) for letting a medical fluid out of the chamber (100), and
- arranging a spike element (14) at the inlet end (101) of the body (10), the spike element (14) forming an inlet (20) for letting a medical fluid into the chamber (100), a connection element (15) being rotatably arranged on the spike element (14) for releasably connecting the drip chamber (1) to a connector (2),
**characterized in**
**that** the spike element (14) is integrally formed with a cap element (13) for closing off the body (10) at the inlet end (101), and wherein the connection element (15) comprises at least one elastically deformable detent element (152) such that the connection element can be slid onto the spike element from a far end of the spike element opposite to the cap element, and along the spike element until the at least one detent element of the connection element snaps into place with a protrusion element (142) of the spike element (14), the protrusion element (142) extending circumferentially about a shaft portion (144) of the spike element (14).

## Patentansprüche

1. Tropfkammer (1) zum Verabreichen von enteraler Ernährung oder eines anderen medizinischen Fluids, umfassend:
- einen Körper (10), der eine Kammer (100) bildet und ein Einlassende (101) und ein Auslassende (103) aufweist,
- ein Spießelement (14), das am Einlassende (101) des Körpers (10) angeordnet ist und einen Einlass (20) zum Zulassen eines medizinischen Fluids in die Kammer (100) bildet,
- einen Auslass (11), der am Auslassende (103) angeordnet und mit einem Schlauch (3) zum Ablassen eines medizinischen Fluids aus der Kammer (100) verbindbar ist, und
- ein Verbindungselement (15), das drehbar am Spießelement (14) angeordnet ist, um die Tropfkammer (1) lösbar mit einem Verbindungsstück (2) zu verbinden,
**gekennzeichnet durch**
ein Kappenelement (13), das mit dem Körper (10) verbunden ist, um den Körper (10) am Einlassende (101) zu verschließen, wobei das Kappenelement (13) einstückig mit dem Spießelement (14) gebildet ist, und wobei das Verbindungselement (15) mindestens ein elastisch verformbares Rastelement (152) umfasst, so dass das Verbindungselement von einem dem Kappenelement gegenüberliegenden entfernten Ende des Spießelements auf das Spießelement und entlang des Spießelements geschoben werden kann, bis das mindestens eine Rastelement des Verbindungselements mit einem Vorsprungselement (142) des Spießelements (14) an seinem Platz einschnappt, wobei sich das Vorsprungselement (142) in Umfangsrichtung um einen Schaftabschnitt (144) des Spießelements (14) erstreckt.

2. Tropfkammer (1) nach Anspruch 1, **dadurch gekennzeichnet, dass** das Kappenelement (13) einstückig mit dem Spießelement (14) durch Spritzgießen gebildet ist.

3. Tropfkammer (1) nach Anspruch 1 oder 2, **dadurch gekennzeichnet, dass** das Verbindungselement (15) als eine Überwurfmutter geformt ist, die eine Öffnung (150) und ein darin angeordnetes Schraubgewinde (151) aufweist.

4. Tropfkammer (1) nach einem der Ansprüche 1 bis 3, **dadurch gekennzeichnet, dass** das Verbindungselement (15) mit dem Spießelement (14) durch eine formschlüssige Verriegelungsverbindung axial verbunden ist.

5. Tropfkammer (1) nach einem der vorhergehenden Ansprüche, **dadurch gekennzeichnet, dass** das Verbindungselement (15) zusammen mit dem Spießelement (14) und dem Kappenelement (13) durch einen generativen Fertigungsprozess hergestellt wird.

6. Tropfkammer (1) nach einem der vorhergehenden Ansprüche, **dadurch gekennzeichnet, dass** das Verbindungselement (15) zusammen mit dem Spießelement (14) und dem Kappenelement (13) durch eine Sintertechnik oder durch eine Zweikomponenten-Spritzgusstechnik hergestellt wird.

7. Tropfkammer (1) nach einem der vorhergehenden Ansprüche, **dadurch gekennzeichnet, dass** das Kappenelement (13) einen Flanschabschnitt (102) umfasst, der auf einem Anlageabschnitt (102) des Körpers (10) aufliegt, um eine fluiddichte Verbindung zwischen dem Kappenelement (13) und dem Körper (10) zu bilden.

8. Tropfkammer (1) nach einem der vorhergehenden Ansprüche, **dadurch gekennzeichnet, dass** das Kappenelement (13) mit dem Körper (10) durch Kleben oder Schweißen verbunden ist.

9. Tropfkammer (1) nach einem der vorhergehenden Ansprüche, **dadurch gekennzeichnet, dass** das Kappenelement (13) eine Öffnung (143) umfasst, die in Fluidverbindung mit einem Strömungskanal (140) des Spießelements (14) steht.

10. Verfahren zum Herstellen einer Tropfkammer (1) zum Verabreichen von enteraler Ernährung oder eines anderen medizinischen Fluids, umfassend:
- Bereitstellen eines Körpers (10), der eine Kammer (100) bildet und ein Einlassende (101) und ein Auslassende (103) aufweist, wobei ein Auslass (11) am Auslassende (103) angeordnet ist und mit einem Schlauch (3) zum Auslassen eines medizinischen Fluids aus der Kammer (100) verbindbar ist, und
- Anordnen eines Spießelements (14) am Einlassende (101) des Körpers (10), wobei das Spießelement (14) einen Einlass (20) bildet, um ein medizinisches Fluid in die Kammer (100) einzulassen, wobei ein Verbindungselement (15) drehbar am Spießelement (14) angeordnet ist, um die Tropfkammer (1) lösbar mit einem Verbindungsstück (2) zu verbinden,
**dadurch gekennzeichnet,**
**dass** das Spießelement (14) einstückig mit einem Kappenelement (13) zum Verschließen des Körpers (10) am Einlassende (101) gebildet ist, und wobei das Verbindungselement (15) mindestens ein elastisch verformbares Rastelement (152) umfasst, so dass das Verbindungselement von einem dem Kappenelement gegenüberliegenden entfernten Ende des Spießelements auf das Spießelement und entlang des Spießelements geschoben werden kann, bis das mindestens eine Rastelement des Verbindungselements mit einem Vorsprungselement (142) des Spießelements (14) an seinem Platz einschnappt, wobei sich das Vorsprungselement (142) in Umfangsrichtung um einen Schaftabschnitt (144) des Spießelements (14) erstreckt.

## Revendications

1. Chambre compte-gouttes (1) permettant d'administrer une nutrition entérale ou un autre fluide médical, comprenant :
- un corps (10) formant une chambre (100) et ayant une extrémité d'entrée (101) et une extrémité de sortie (103),
- un élément de pointe (14) agencé au niveau de l'extrémité d'entrée (101) du corps (10) et formant une entrée (20) pour laisser entrer un fluide médical dans la chambre (100),
- une sortie (11) agencée au niveau de l'extrémité de sortie (103) et pouvant être reliée à un tube (3) pour laisser sortir un fluide médical hors de la chambre (100), et
- un élément de liaison (15) agencé de manière rotative sur l'élément de pointe (14) pour relier de manière amovible la chambre compte-gouttes (1) à un connecteur (2),
**caractérisée par**
un élément de capuchon (13) relié au corps (10) pour fermer le corps (10) au niveau de l'extrémité d'entrée (101), l'élément de capuchon (13) étant formé d'un seul tenant avec l'élément de pointe (14), et l'élément de liaison (15) comprenant au moins un élément d'encliquetage (152) élastiquement déformable de telle sorte que l'élément de liaison peut être coulissé sur l'élément de pointe à partir d'une extrémité éloignée de l'élément de pointe à l'opposé de l'élément de capuchon, et le long de l'élément de pointe jusqu'à ce que l'au moins un élément d'encliquetage de l'élément de liaison s'emboîte en place avec un élément saillant (142) de l'élément de pointe (14), l'élément saillant (142) s'étend circonférentiellement autour d'une partie d'arbre (144) de l'élément de pointe (14).

2. Chambre compte-gouttes (1) selon la revendication 1, **caractérisée en ce que** l'élément de capuchon (13) est formé d'un seul tenant avec l'élément de pointe (14) par moulage par injection.

3. Chambre compte-gouttes (1) selon la revendication 1 ou 2, **caractérisée en ce que** l'élément de liaison (15) est profilé en un écrou d'accouplement ayant une ouverture (150) et un filetage de vis (151) agencé en son sein.

4. Chambre compte-gouttes (1) selon l'une des revendications 1 à 3, **caractérisée en ce que** l'élément de liaison (15) est fixé axialement à l'élément de pointe (14) par une liaison à verrouillage positif.

5. Chambre compte-gouttes (1) selon l'une des revendications précédentes, **caractérisée en ce que** l'élément de liaison (15) est fabriqué conjointement avec l'élément de pointe (14) et l'élément de capuchon (13) par un processus de fabrication génératif.

6. Chambre compte-gouttes (1) selon l'une des revendications précédentes, **caractérisée en ce que** l'élément de liaison (15) est fabriqué conjointement avec l'élément de pointe (14) et l'élément de capuchon (13) par une technique de frittage ou par une technique de moulage par injection à deux composants.

7. Chambre compte-gouttes (1) selon l'une des revendications précédentes, **caractérisée en ce que** l'élément de capuchon (13) comprend une partie de bride (102) reposant sur une partie de butée (102) du corps (10) pour former une liaison étanche aux fluides entre l'élément de capuchon (13) et le corps (10).

8. Chambre compte-gouttes (1) selon l'une des revendications précédentes, **caractérisée en ce que** l'élément de capuchon (13) est relié au corps (10) par collage ou soudage.

9. Chambre compte-gouttes (1) selon l'une des revendications précédentes, **caractérisée en ce que** l'élément de capuchon (13) comprend une ouverture (143) qui est en communication fluidique avec un canal d'écoulement (140) de l'élément de pointe (14).

10. Procédé de production d'une chambre compte-gouttes (1) permettant d'administrer une nutrition entérale ou un autre fluide médical, comprenant :
- la fourniture d'un corps (10) formant une chambre (100) et ayant une extrémité d'entrée (101) et une extrémité de sortie (103), une sortie (11) étant agencée au niveau de l'extrémité de sortie (103) et pouvant être reliée à un tube (3) pour laisser sortir un fluide médical hors de la chambre (100), et
- l'agencement d'un élément de pointe (14) au niveau de l'extrémité d'entrée (101) du corps (10), l'élément de pointe (14) formant une entrée (20) pour laisser entrer un fluide médical dans la chambre (100), un élément de liaison (15) étant agencé de manière rotative sur l'élément de pointe (14) pour relier de manière amovible la chambre compte-gouttes (1) à un connecteur (2),
**caractérisé en**
l'élément de pointe (14) est formé d'un seul tenant avec un élément de capuchon (13) pour fermer le corps (10) au niveau de l'extrémité d'entrée (101), et dans lequel l'élément de liaison (15) comprend au moins un élément d'encliquetage (152) élastiquement déformable de telle sorte que l'élément de liaison peut être coulissé sur l'élément de pointe à partir d'une extrémité éloignée de l'élément de pointe à l'opposé de l'élément de capuchon, et le long de l'élément de pointe jusqu'à ce que l'au moins un élément d'encliquetage de l'élément de liaison s'emboîte en place avec un élément saillant (142) de l'élément de pointe (14), l'élément saillant (142) s'étend circonférentiellement autour d'une partie d'arbre (144) de l'élément de pointe (14).
